# EUROPÄISCHE PATENTSCHRIFT

(11) **EP 2 457 097 B1**
(45) Veröffentlichungstag und Bekanntmachung des Hinweises auf die Patenterteilung: **09.09.2015**
(21) Anmeldenummer: 10735203.1
(22) Anmeldetag: 15.07.2010
(51) Int. Cl.: G01N 33/74, A61M 1/36, B01J 20/32

(54) **ADSORBENS ZUR ADSORPTION VON HEPCIDIN**
ADSORBENTS FOR THE ADSORPTION OF HEPCIDIN
ADSORBANT POUR L'ADSORPTION D'HEPCIDINE

(30) Priorität: 20.07.2009 DE 102009034150
(43) Veröffentlichungstag der Anmeldung: 30.05.2012
(73) Patentinhaber: Fresenius Medical Care Deutschland GmbH, 61352 Bad Homburg (DE)
(72) Erfinder: PASSLICK-DEETJEN, Jutta, 35392 Giessen (DE); HOFMANN, Wolfgang, 60487 Frankfurt (DE)
(74) Vertreter: Weiß, Stefan
(86) Internationale Anmeldenummer: PCT/EP2010/004303
(87) Internationale Veröffentlichungsnummer: WO 2011/009555

(56) Entgegenhaltungen:
- EP-A1- 1 481 700
- WO-A1-03/090924
- WO-A1-2006/042507
- WO-A1-2009/044284
- WO-A1-2009/058797
- PESLOVA GABRIELA ET AL: "Hepcidin, the hormone of iron metabolism, is bound specifically to alpha-2-macroglobulin in blood" BLOOD, Bd. 113, Nr. 24, Juni 2009 (2009-06), Seiten 6225-6236, XP002604556 ISSN: 0006-4971

## Beschreibung

Die Erfindung betrifft eine Vorrichtung und eine Methode zur Abreicherung von Hepcidin aus Blut zur Behandlung von Eisenmangel, insbesondere von anämischen Patienten, die an chronischer Nierenerkrankung leiden mit dem Ziel die Eisenverfügbarkeit im Organismus zu erhöhen und damit die Therapie der vorhandenen Anämie zu verbessern.

Humanes Hepcidin wird als Prepropeptid mit 84 Aminosäuren codiert. Davon codieren 24 N-terminale Aminosäuren für eine auf das endoplasmatische Retikulum gerichtete Signalsequenz, 35 Aminosäuren bilden die Proregion und die 25 C-terminalen Aminosäuren bilden die eigentliche bioaktive Sequenz.

Die C-terminale Region, auch Hecidin25 genannt, regelt hormonell den Eisenhaushalt des Körpers. Im Körper werden auch häufig N-terminale Abbauprodukte des Hepcidin25, insbesondere das 20 Aminosäuren unfassende Hepcidin20 und das 22 Aminosäuren unfassende Hepcidin22 angetroffen. Mutationen im Hepcidingen sind mit einer schweren juvenilen Hemochromatose, einer Überladung der Zellen mit Eisen, assoziiert. Im Gegensatz dazu kann Hepcidin in Anwesenheit inflammatorischer Stimuli überexprimiert werden. Die Stimulation wird durch eine Ausschüttung des inflammatorischen Interleukins IL-6 ausgelöst. Daher kann eine starke Korrelation zwischen chronischer Entzündung, wie insbesondere bei chronischer Nierenerkrankung (CKD), Hepcidinexpression und durch Eisenmangel ausgelöster Anämie beobachtet werden.

Hepcidin hindert die Abgabe von Eisen durch die Zellen, indem das Eisenexportprotein Ferroportin abgebaut wird. Über Ferroportin wird Eisen aus der Zelle ins Plasma ausgeschleust. Im gesunden Organismus bewirkt eine Abwesenheit von Hepcidin daher eine erhöhte Ausschleusung von Eisen aus den Zellen und einem überladenen Eisenspiegel im Blutplasma. In chronisch inflammatorischen Patienten mit einem erhöhtem Hepcidinspiegel findet eine Akkumulation des Eisens in den Zellen statt und dies führt zu einem Mangel an transferringebundenem Eisen im Blutplasma. In Patienten mit chronischem Nierenversagen tritt dieser Effekt noch verstärkt durch eine mangelnde Elimination des Hepcidins aus dem Blutspiegel über die Nieren auf. Ein zu geringer Eisenspiegel im Blutplasma ist eng mit dem Krankheitsbild der Anämie verbunden, weil für eine effektive Erythropoese, einer Bildung roter Blutkörperchen, eine ausreichende Eisenversorgung gewährleistet sein muss. Damit stellt ein Mangel in der geeigneten Versorgung des Blutplasmas mit Eisen nicht nur in sich einen möglichen Grund für Anämie dar, sondern verhindert darüber hinaus auch eine erfolgreiche medikamentöse Behandlung der Anämie, beispielsweise mit EPO.

Patienten, die an chronischem Nierenversagen leiden, leiden daher besonders häufig an durch Eisenmangel verursachter Anämie, weil durch den ständigen inflammatorischen Zustand die Hepcidinausschüttung erhöht ist, aufgrund des Nierenversagens zusätzlich aber auch die effektive Elimination des Hepcidins beeinträchtigt ist.

WO2009/058797, WO08/097461 und WO2009/044284 beschreiben einen Hepcidinbindenden Antikörper und die Behandlung anämischer Patienten mittels medikamentöser systemischer Verabreichung des Antikörpers. Eine systemische Antikörpertherapie versetzt den Körper neben den typischen Nebenwirkungen, wie Fieber, Schüttelfrost, Juckreiz, Schwitzen, Blutdruckabfall, Infektionsneigung, Wassereinlagerungen, die häufig durch eine sekundäre Immunreaktion gegen die Antikörper selbst ausgelöst werden, zusätzlich in einen inflammatorischen Zustand, der wie oben beschrieben die Hepcidinproduktion fördert.

Es ist Aufgabe des Patents Mittel und Verfahren zur Abreicherung des Hepcidins im Blutplasma bereitzustellen, die die Nachteile einer medikamentösen Antikörpertherapie vermeidet.

Gelöst wird diese Aufgabe erfindungsgemäß durch die unabhängigen Ansprüche. Teilaufgaben werden in vorteilhafter Weise durch die abhängigen Ansprüche gelöst.

Im Rahmen der Erfindung findet die Abreicherung von Hepcidin durch die Adsorption von Hepcidin in einem extrakorporalen Kreislauf statt. Hierzu wird dem Patienten kontinuierlich Blut entnommen, das entnommene Blut über ein Adsorptionsmittel geleitet, das Hepcidin bindet und das hepcidinabgereicherte Blut dem Patienten rückgeführt. Zu diesem Zweck wird erfindungsgemäß eine Vorrichtung zur Verfügung gestellt die einen extrakorporalen Kreislauf ermöglicht und eine Abreicherungseinheit zur Abreicherung von Hepcidin enthält. Die Abreicherungseinheit besteht aus einem Gehäuse und einem darin enthaltenen Adsorbens, das Hepcidin spezifisch bindet. Das erfindungsgemäße Adsorbens besteht aus einer Festphasenmatrix, an die ein spezifisch Hepcidin-bindender Ligand kovalent gekoppelt ist.

Die Erfindung wird im Folgenden mit Bezug auf begleitende Zeichnungen beispielhaft beschrieben. Es zeigt:

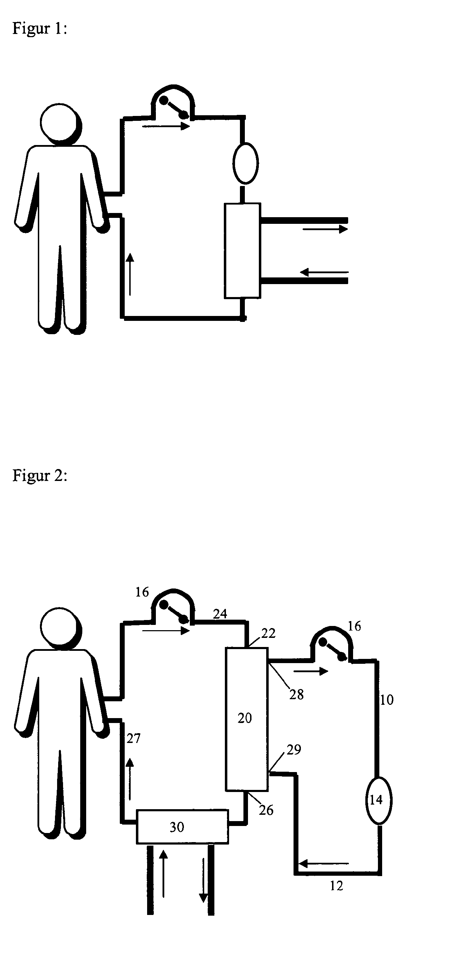

Fig. 1 ein schematisches Blockschaltbild einer bevorzugten Ausführungsform einer erfindungsgemäßen Vorrichtung mit ersterLeitungseinrichtung 10, zweiter Leitungseinrichtung 12, Abreicherungseinheit 14 und Körperflüssigkeitsfördereinrichtung 16 und optional einem Hämodialysator 30.
Fig.2: ein schematisches Blockschaltbild einer weiteren bevorzugten Ausführungsform einer erfindungsgemäßen Vorrichtung mit erster Leitungseinrichtung 10, zweiter Leitungseinrichtung 12, Abreicherungseinheit 14, Flüssigkeitsfördereinrichtung 16, Plasmafilter 20, dritter Leitungseinrichtung 24, vierter Leitungseinrichtung 27, Flüssigkeitszuführeingang 22 der ungefilterten Seite, Flüssigkeitsabführausgang 26 der ungefilterten Seite, Flüssigkeitsabführausgang 28 der gefilterten Seite, Flüssigkeitszuführeingang 29 der gefilterten Seite und optional einem Hämodialysator 30.

Eine mögliche medizinische Vorrichtung zur Etablierung eines extrakorporalen Kreislauf zur Abreicherung von Hepcidin aus Vollblut umfasst daher erfindungsgemäß eine erste (10) und eine zweite (12) mit dem Patienten (P) verbindbare Leitungseinrichtung, eine zwischen der ersten (10) und der zweiten (12) Leitungseinrichtung angeordnete und damit flüssigkeitsdicht verbundene Abreicherungseinheit enthaltend ein Adsorbens nach Anspruch 1 (14) und zumindest eine steuerbare, in der ersten (10) und/oder zweiten (12) Leitungseinrichtung angeordnete Körperflüssigkeitsfördereinrichtung (16), worin mittels der zumindest einen Körperflüssigkeitsfördereinrichtungen (16) ein steuerbarer Durchfluß der Körperflüssigkeit durch die Leitungseinrichtungen (10, 12) und die Körperflüssigkeitsbestrahlungseinrichtung (14) erzeugbar ist, wobei die erste Leitungseinrichtung (10) ausgelegt ist, die dem Patienten (P) entnommene Körperflüssigkeit der Abreicherungseinheit (14) kontinuierlich zuzuführen und die zweite Leitungseinrichtung (12) ausgelegt ist, die bestrahlte Körperflüssigkeit dem Patienten (P) kontinuierlich zuzuführen.

Eisenmangel betrifft besonders häufig Patienten mit chronischem Nierenversagen. Diese Patienten werden häufig zum Ersatz der Nierentätigkeit durch Hämodialyse behandelt. In der Hämodialyse wird ebenfalls ein extrakorporaler Kreislauf etabliert, bei dem dem Patienten kontinuierlich Blut entnommen wird, das Blut über eine semipermeable Membran geleitet wird, um auf diese Weise dem Blut toxische Stoffe sowie Flüssigkeit per Dialyse an der Membran zu entziehen und das gereinigte Blut dem Patienten rückgeführt. Es bietet sich daher im Rahmen der Erfindung an die beiden Therapien vorteilhaft zu verbinden, indem die medizinische Vorrichtung, die zum Aufbau des extrakorporalen Kreislaufs dient, sowohl ein Hämodialysator als auch eine Abreicherungseinheit für Hepcidin enthalten ist. Auf diese Weise kann eine Abreicherung des Hepcidin mittels einer Adsorption gleichzeitig mit der Hämodialyse erfolgen.

Es ist bekannt, dass die Adsorption von Stoffen aus dem Blutplasma häufig effektiver ist als die Adsorption aus dem Vollblut. Eine alternative erfindungsgemäße Vorrichtung trennt daher über einen Sekundärkreislauf Blut in zellfreies Blutplasma und eine Zellsuspension. Die Adsorption des Hepcidin aus dem Blutplasma erfolgt dann mittels der Abreicherungseinheit im Sekundärkreislauf. Das abgereicherte Blutplasma wird aus dem Sekundärkreislauf umgehend dem Primärkreislauf rückgeführt.

Eine entprechende medizinische Vorrichtung umfasst zusätzlich zu der vorhergehend beschriebenen medizinischen Vorrichtung weiterhin einen Plasmafilter (20) mit einer ungefilterten Seite und einer gefilterten Seite aufweist, wobei die ungefilterte Seite von der gefilterten Seite durch zumindest ein Filtermaterial getrennt ist, wobei
- ein Flüssigkeitszuführeingang (22) der ungefilterten Seite mit einer dritten (24) mit dem Patienten verbindbaren Leitungseinrichtung verbunden ist,
- ein Flüssigkeitsabführausgang (26) der ungefilterten Seite mit einer vierten (27) mit dem Patienten verbindbaren Leitungseinrichtung verbunden ist,
- ein Flüssigkeitsabführausgang (28) der gefilterten Seite mit der ersten Leitungseinrichtung (10) verbunden ist, und
- ein Flüssigkeitszuführeingang (29) der gefilterten Seite mit der zweiten Leitungseinrichtung (12) verbunden ist.

Das Filtermaterial des Plasmafilters ist vorteilhaft so gestaltet, dass Blutzellen im Primärkreislauf verbleiben wogegen Makromoleküle den Plasmafilter passieren können. Der Cut-off des Plasmafilters liegt daher bevorzugt zwischen 1000 und 5000 kDa.

Hepcidin wird in Blut oder Blutplasma überwiegend in gebundener Form vorgefunden. Hepcidin bindet mit hoher Affinität an Alpha-2-Makroglobulin mit einer Dissoziationskonstanten von 177 nM. Das physiologisch aktive Hepcidin25 und seine Fragmente besitzen ein Molekulargewicht von weniger als 3kDa. Trotzdem wird Hepcidin bei der Hämodialyse durch seine Bindung an das wesentlich größere Alpha-2-Makroglobulin (750 kDa), das die Dialysemembran nicht passieren kann, nur unzureichend eliminiert. Um eine effektive Adsorption von Hepcidin aus Blut oder Blutplasma zu gewährleisten, muss die Affinität zu dem in der Abreicherungseinheit verwendeten Adsorbens mindestens in der gleichen Größenordnung wie die Affinität von Hepcidin zu Alpha-2-Makroglobulin liegen. Dies wird durch ein Adsorbens mit an die Festphase gebundenen Liganden mit einer Affinität zu Hepcidin25 mit einer Dissoziationskonstanten von kleiner als 200 nM erreicht. Bevorzugt ist die Dissoziationskonstante kleiner als 50 nM, mehr bevorzugt kleiner als 5 nM und besonder bevorzugt kleiner als 1 nM.

Hepcidin bindende Liganden im Sinne der Erfindung können monoklonale Antikörper und deren Fragmente sein. Alternative Liganden im Sinne der Erfindung können Hepcidin bindende Peptide sein. Solche Peptide können von der Hepcidin bindenden Peptidsequenz des Alpha-2-Makroglobulins abgeleitet sein. Geeignete Liganden im Sinne der Erfindung sind beispielhaft die Antikörper, die in den Patentanmeldungen WO2009/058797, WO08/097461 und WO2009/044284 offenbart wurden.

Das Adsorbens umfasst an eine Festphase kovalent gebundene Liganden. Die Festphase kann auf organischem oder anorganischem Material basieren, bevorzugt ist die Festphase aus organischem Material. Die Festphase ist idealerweise Vollblut-verträglich. Ein Vollblutverträgliches Adsorbens ermöglicht die Adsorption in einem extrakorporalen Kreislauf ohne eine Auftrennung in Blutplasma und Zellsuspension. Auf diese Weise kann eine medizinische Vorrichtung verwendet werden, die ausschließlich einen Primärkreislauf aufweist, im Gegensatz zu der ebenfalls offenbarten aufwendigeren Alternative einer medizinischen Vorrichtung, die neben dem Primärkreislauf einen Sekundärkreislauf beinhaltet.

Vollblutadsorbentien bestehen aus Teilchen, welche so groß sind, dass sie Zwischenräume bilden, in denen sich die Blutzellen bewegen können. Ferner weisen die Teilchen Poren auf, welche zu einer inneren Oberfläche führen. Diese Poren weisen eine ausreichende Größe auf, dass sogar Makromoleküle in sie eindringen können. Die Poren sind jedoch so klein gewählt, dass das Eindringen von Blutzellen ausgeschlossen ist. Die Blutzellen haben somit nur Kontakt zur äußeren Oberfläche der Teilchen. Ferner müssen diese Teilchen gemäß EP 0 424 698 möglichst sphärisch und unaggregiert sein, um eine "glatte" sowie auch eine inerte Außenseite aufzuweisen, so dass die Thrombozyten daran abgleiten.

Die Abreicherungseinheit besteht vorteilhafterweise aus einem bevorzugt starren Gehäuse befüllt mit dem teilchenförmigen Adsorbens. Durch feinmaschige Siebe am ein und Auslass des Bluts oder des Blutplasmas werden die Teilchen im inneren des Gehäuses gehalten und so der Übertritt in das Blut des Patienten vermieden.

Das poröse organische Trägermaterial des erfindungsgemäßen Adsorbens weist eine mittlere Porengröße bzw. einen mittleren Porendurchmesser mit einem Wert von ≤ 1,5 µm, vorzugsweise µ 1,0 µm, auf, wobei maximal 50% des Porenvolumens in Poren mit einer Porengröße von > 1,5 µm vorliegen. Die maximale mittlere Porengröße von1,5 µm ist durch die Größe der kleinsten Blutzellen bedingt, welche einen Durchmesser von etwa 2 µm aufweisen. Bei einer, wie gemäß der vorliegenden Erfindung definierten, maximalen mittleren Porengröße ist sichergestellt, dass im Wesentlichen keine Blutzellen in die Poren eindringen können. Auf der anderen Seite sollen die Poren groß genug sein um die abzureichernden Substanzen aufnehmen zu können. Im Falle von Hepcidin sollen die Poren nicht nur das kleine Hepcidin aufnehmen können, sonderen darüber hinaus auch das wesentlich größer Hepcidin-bindende Alpha-2-Makroglobulin. Die Poren müssen also geeignet sein Makromoleküle mit einem Molekulargewicht von 750 kDa aufzunehmen. Aus diesem Grunde besitzen die Poren bevorzugt eine mittlere Größe von mindestens 0,05 µm, bevorzugt ein Größe von mindestens 0,1 µm und besonders bevorzugt ein Größe von mindestens 0,3 µm.

Erfindungsgemäß kann die mittlere Porengröße durch Quecksilberintrusion (Quecksilberporosimetrie) analog DIN 66 133 bestimmt werden. Das Verfahren beruht auf der Messung des in einen porösen Feststoff eingepressten Quecksilbervolumens in Abhängigkeit vom angewandten Druck. Eine nichtbenetzende Flüssigkeit wie Quecksilber dringtnur unter Druck in ein poröses System ein. Der aufzuwendende Druck ist umgekehrt proportional zur lichten Weite der Porenöffnungen. Dabei werden Poren erfasst, in die bei dem angewendeten Druck Quecksilber eindringen kann. Für zylindrische Poren ist der Zusammenhang zwischen Porenradius rp und Druck p durch die Washbum-Gleichung gegeben: wobei s die Oberflächenspannung des Quecksilbers [N/m] und J der Kontaktwinkel des Quecksilbers auf der Probe, gemessen durch die flüssige Phase ist. In Abweichung zur DIN Norm 66 133 wird für den Kontaktwinkel der feste Wert141,3° eingesetzt und es werden bei der Bestimmung der mittleren Porengröße die Zwischenkornvolumina nicht mitgerechnet.

Ferner weisen die Adsorbensteilchen vorzugsweise eine Korngröße von 50 bis 500 µm, mehr bevorzugt von100 bis 300 µm, auf. Bei einem Einsatz in einem extrakorporalen Kreislauf mit Vollblut sollten die Teilchen des Adsorbens eine Korngröße von mindestens 50 µm aufweisen, da die im Vollblut vorhandenen größten Blutkörperchen bzw. Blutzellen einen Durchmesser von 20 µm besitzen. Somit muss das Sieb, welches das Adsorbens zurückhält, eine Maschenweite von mindestens 25 µm, vorzugsweise mindestens 40 µm, haben, damit alle Blutzellen durchgelassen werden. Ferner ist in den mit Adsorbensteilchen einer Größe von 50 µm und mehr gepackten Säulen der Zwischenraum zwischen denTeilchen für den Durchlaß von Blutzellen ausreichend. Bei der Auswahl des Siebmaterials ist ferner darauf zu achten, dass die Maschenweite stets kleiner ist, als der Durchmesser der kleinsten Adsorberteilchen.

Das erfindungsgemäße Adsorbens umfasst bevorzugt ein poröses teilchenförmiges Trägermaterial. Nach dieser Ausführungsform sind Trägermaterialien geeignet, wie beispielsweise,Kohlenhydrate, Sepharose oder organische Trägermaterialien, wie Copolymere von Acrylaten oder Methacrylaten sowie Polyamiden. Vorzugsweise besteht das Trägermaterial aus von (Meth)-Acrylsäureestern und/oder -amiden abgeleiteten Copolymeren. Diese weisen vorzugsweise Epoxidgruppen auf. Unter dem Begriff "(Meth)-Acryl" sind sowohl die entsprechenden Acryl- als auch Methacrylverbindungen zu verstehen.

Der Ligand kann mit jeder im Stand der Technik bekannten und üblichen Methode kovalent an das Trägermaterial gebunden werden. Übliche Methoden sind die z.B. Bindungen über eine Epoxyreaktion, eine Ni-Base Reaktion, eine Kondensationsreaktion mit einem Carbodiimid, eine Reaktion mit Esteraktivierung und Kreuzvernetzungsreaktionen mit einem Glutaraldehyd. Neben den genannten üblichen Methoden zur Immobilisierung von Liganden auf Festphasen gibt es verschiedene Anbieter von Festphasensystemem (z.B. Eupergit, Toyopearl), die eine bestimmte durch die Eigenschaften der Festphase vorgegebene oder besonders vorteilhafte Bindung an die Festphase vorgeben.

### Beispiel 1:

Eine aufgereinigte Fraktion monoklonarer Hepcidin25 bindender Antikörper des Typs IgG wurde bei einem pH von 7,5 in PBS in einer Konzentration von 3 mg/ml gelöst. Zu 5 ml der Lösung wurde 1 g Oxiranacryl-Beads (Eupergit C, Röhm GmbH&Co KG) gegeben. Die Suspension wurde für 48 Stunden bei Raumtemperatur im Dunkels gelagert und dann mit weiteren 3 ml PBS verdünnt. Nach der Abtrennung der Beads vom Überstand, konnte im Überstand nur noch eine Konzentration von weniger als 50 µg/ml festgestellt werden. Das entstandene Adsorbens wurde mit PBS bei pH 7,5 gewaschen. Das Adsorbens wurde mit 10 ml einer 10% wässrigen Ethanolaminlösung bei pH 9 und einer Temperatur von 4°C 12 Stunden gerührt um verbleibenden Oxirangruppen abzureagieren. Das Adsorbens wurde abschließend wiederum mit PBS bei einem pH von 7,5 gewaschen.

## Patentansprüche

1. Adsorbens zur Verwendung in einem extrakorporalen Kreislauf zur Absenkung der Konzentration von Hepcidin in Blut oder Blutplasma umfassend eine Matrix und einem kovalent an die Matrix gebundenen Liganden, der Hepcidin mit einer Affinität bindet, die eine Dissoziationskonstante KD von kleiner als 200 nM besitzt.

2. Adsorbens nach Anspruch 1 **dadurch gekennzeichnet, dass** der Ligand ein monoklonaler Antikörper oder ein Antikörperfragment ist.

3. Adsorbens nach Anspruch 2 **dadurch gekennzeichnet, dass** der Antikörper oder das Antikörperfragment humanisiert sind.

4. Adsorbens nach Anspruch 1 **dadurch gekennzeichnet, dass** der Ligand ein Peptid ist, das eine mindestens 80 prozentige Homologie zur Peptidsequenz der Bindungsregion des alpha 2 Makroglobulins aufweist.

5. Adsorbens nach einem der Ansprüche 1 bis 4 **dadurch gekennzeichnet, dass** die Matrix aus im Wesentlichen sphärischen, nicht aggregierten, porösen Teilchen besteht.

6. Adsorbens nach einem der Ansprüche 1 bis 5 **dadurch gekennzeichnet, dass** die Matrix Poren einer mittleren Porengröße von weniger als 1,5 µm und mehr als 0,1 µM aufweist.

7. Adsorbens nach einem der Ansprüche 1 bis 6 **dadurch gekennzeichnet, dass** die Matrix aus einem organischen Polymer aufgebaut ist.

8. Medizinische Vorrichtung zur Abreicherung von unerwünschten Substanzen aus Blut oder Blutplasma umfassend eine erste (10) und eine zweite (12) mit dem Patienten (P) verbindbare Leitungseinrichtung,eine zwischen der ersten (10) und der zweiten (12) Leitungseinrichtung angeordnete und damit flüssigkeitsdicht verbundene Abreicherungseinheit (14) enthaltend ein Adsorbens nach einem der Ansprüche 1 bis 7 und zumindest eine steuerbare, in der ersten (10) und/oder zweiten (12) Leitungseinrichtung angeordnete Körperflüssigkeitsfördereinrichtung (16), worin mittels der zumindest einen Körperflüssigkeitsfördereinrichtungen (16) ein steuerbarer Durchfluß der Körperflüssigkeit durch die Leitungseinrichtungen (10, 12) und die Abreicherungseinheit (14) erzeugbar ist, wobei die erste Leitungseinrichtung (10) ausgelegt ist, die dem Patienten (P) entnommene Körperflüssigkeit der Abreicherungseinheit (14) kontinuierlich zuzuführen und die zweite Leitungseinrichtung (12) ausgelegt ist, die bestrahlte Körperflüssigkeit dem Patienten (P) kontinuierlich zuzuführen.

9. Medizinische Vorrichtung nach Anspruch 8 **dadurch gekennzeichnet dass** sie einen Hämodialysator enthält.

10. Medizinische Vorrichtung nach einem der Ansprüche 8 bis 9, welche weiterhin einen Filter (20) mit einer ungefilterten Seite und einer gefilterten Seite aufweist, wobei die ungefilterte Seite von der gefilterten Seite durch zumindest ein Filtermaterial getrennt ist, wobei - ein Flüssigkeitszuführeingang (22) der ungefilterten Seite mit einer dritten (24) mit dem Patienten verbindbaren Leitungseinrichtung verbunden ist,
- ein Flüssigkeitsabführausgang (26) der ungefilterten Seite mit einer vierten (27) mit dem Patienten verbindbaren Leitungseinrichtung verbunden ist,
- ein Flüssigkeitsabführausgang (28) der gefilterten Seite mit der ersten Leitungseinrichtung (10) verbunden ist, und
- ein Flüssigkeitszuführeingang (29) der gefilterten Seite mit der zweiten Leitungseinrichtung (12) verbunden ist.

11. Adsorbens nach einem der Ansprüche 1 bis 7 zur Verwendung in der Behandlung von Eisenmangel in einem anämischen Patienten, wobei der anämische Patient an chronischem Nierenversagen leidet.

12. Adsorbens nach Anspruch 11 **dadurch gekennzeichnet, dass** die Behandlung gleichzeitig mit einer Hämodialyse durchgeführt wird.

## Claims

1. An adsorbent for use in an extracorporeal circulation for reducing the hepcidin concentration in blood or blood plasma, comprising a matrix and a ligand which is bound covalently to the matrix and which binds hepcidin with an affinity having a dissociation constant KD of less than 200 nM.

2. The adsorbent according to Claim 1, **characterized in that** the ligand is a monoclonal antibody or an antibody fragment.

3. The adsorbent according to Claim 2, **characterized in that** the antibody or the antibody fragment is humanized.

4. The adsorbent according to Claim 1, **characterized in that** the ligand is a peptide having an at least 80 percent homology with the peptide sequence of the binding region of the alpha-2-macroglobulin.

5. The adsorbent according to any one of Claims 1 to 4, **characterized in that** the matrix consists of essentially spherical non-aggregated porous particles.

6. The adsorbent according to any one of Claims 1 to 5, **characterized in that** the matrix has pores with an average pore size of less than 1.5 µm and more than 0.1 µm.

7. The adsorbent according to any one of Claims 1 to 6, **characterized in that** the matrix is constructed of an organic polymer.

8. A medical device for reducing the concentrations of unwanted substances in blood or blood plasma, comprising a first line device (10) and a second line device (12), which can be connected to the patient (P), a depletion unit (14), which is arranged between the first line device (10) and the second line device (12) and is thus fluid-tight and contains an adsorbent according to any one of Claims 1 to 7, and at least one controllable body fluid pumping device (16) arranged in the first line device (10) and/or the second line device (12), wherein a controllable flow of body fluid through the line devices (10, 12) and the depletion unit (14) can be created by means of the at least one body fluid pumping device (16), wherein the first line device (10) is designed to continuously supply body fluid taken from the patent (P) to the depletion unit (14), and the second line device (12) is designed to continuously supply the irradiated body fluid to the patient (P).

9. The medical device according to Claim 8, **characterized in that** it includes a hemodialyzer.

10. The medical device according to any one of Claims 8 to 9, also having a filter (20) with an unfiltered side and a filtered side, wherein the unfiltered side is separated from the filtered side by at least one filter material, wherein
- a liquid supply inlet (22) on the unfiltered side is connected to a third line device (24), which can in turn be connected to the patient,
- a liquid discharge outlet (26) on the unfiltered side is connected to a fourth line device (27), which can be connected to the patient,
- a liquid discharge outlet (28) on the filtered side is connected to the first line device (10), and
- a liquid supply inlet (29) on the filtered side is connected to the second line device (12).

11. The adsorbent according to any one of Claims 1 to 7 for use in the treatment of iron deficiency in an anemic patient, wherein the anemic patient is suffering from chronic renal failure.

12. The adsorbent according to Claim 11, **characterized in that** the treatment is performed simultaneously with hemodialysis.

## Revendications

1. Adsorbant destiné à être employé dans un circuit extracorporel pour baisser la concentration d'hepcidine dans du sang ou du plasma sanguin comprenant une matrice et un covalent aux ligands liés à la matrice, qui lie l'hepcidine à une affinité qui possède une constante de dissociation KD inférieure à 200 nM.

2. Adsorbant selon la revendication 1, **caractérisé en ce que** le ligand est un anticorps monoclonal ou un fragment d'anticorps.

3. Adsorbant selon la revendication 2, **caractérisé en ce que** l'anticorps ou le fragment d'anticorps est humanisé.

4. Adsorbant selon la revendication 1, **caractérisé en ce que** le ligand est un peptide qui présente une homologie d'au moins 80 pourcents à la séquence peptidique de la région de liaison de l'alpha 2 macroglobuline.

5. Adsorbant selon l'une des revendications 1 à 4, **caractérisé en ce que** la matrice est composée de particules essentiellement sphériques, non agrégées et poreuses.

6. Adsorbant selon l'une des revendications 1 à 5, **caractérisé en ce que** la matrice présente des pores d'une taille de pore moyenne inférieure à 1,5 µm et supérieure à 0 ,1 µm.

7. Adsorbant selon l'une des revendications 1 à 6, **caractérisé en ce que** la matrice est constituée d'un polymère organique.

8. Dispositif médical destiné à l'appauvrissement de substances indésirables de sang ou de plasma sanguin comprenant un premier (10) et un deuxième (12) dispositif de lignes pouvant être relié au patient (P), un dispositif d'appauvrissement (14) disposé entre le premier (10) et le deuxième (12) dispositif de lignes et relié en étanchéité fluidique à ceux-ci, comprenant un adsorbant selon l'une des revendications 1 à 7 et au moins un dispositif de transport de liquide corporel (16) pouvant être commandé, disposé dans le premier (10) et/ou le deuxième (12) dispositif de lignes, où un débit pouvant être commandé de liquide corporel peut être produit par les dispositifs de lignes (10, 12) et l'unité d'appauvrissement (14), au moyen de l'au moins un dispositif de transport de liquide corporel (16), sachant que le premier dispositif de lignes (10) est conçu pour amener en continu à l'unité d'appauvrissement (14) le liquide corporel prélevé au patient (P), et le deuxième dispositif de lignes (12) est conçu pour amener en continu au patient (P) le liquide corporel traité par irradiation.

9. Dispositif médical selon la revendication 8, **caractérisé en ce qu'**il contient un hémodialyseur.

10. Dispositif médical selon l'une des revendications 8 à 9, présentant en outre un filtre (20) avec un côté non filtré et un côté filtré, sachant que le côté non filtré est séparé du côté filtré par au moins un matériau filtrant, sachant
- qu'une admission de liquide (22) du côté non filtré est reliée à un troisième (24) dispositif de lignes pouvant être relié au patient,
- qu'une évacuation de liquide (26) du côté non filtré est reliée à un quatrième (27) dispositif de lignes pouvant être relié au patient,
- qu'une évacuation de liquide (28) du côté filtré est reliée au premier dispositif de lignes (10), et
- qu'une admission de liquide (29) du côté filtré est reliée au second dispositif de lignes (12).

11. Adsorbant selon l'une des revendications 1 à 7 destiné à être utilisé dans le traitement de la carence en fer chez un patient anémique, sachant que le patient anémique souffre d'insuffisance rénale chronique.

12. Adsorbant selon la revendication 11, **caractérisé en ce que** le traitement est effectué parallèlement à une hémodialyse.
